# EUROPEAN PATENT SPECIFICATION

(11) **EP 2 113 262 B1**
(45) Date of publication and mention of the grant of the patent: **06.11.2013**
(21) Application number: 09159131.3
(22) Date of filing: 29.04.2009
(51) Int. Cl.: A61L 31/00, A61L 27/36

(54) **A Tissue Repair Implant**
Gewebereparaturimplantat
Implant pour réparation tissulaire

(30) Priority: 29.04.2008 US 48812 P
(43) Date of publication of application: 04.11.2009
(73) Proprietor: Proxy Biomedical Limited, Co Galway (IE)
(72) Inventor: Gingras, Peter, 00, Galway (IE); Voskerician, Gabriela, Beachwood, OH 44122 (US); White, Michael, 00, Sligo (IE)
(74) Representative: O'Neill, Brian

(56) References cited:
- WO-A-99/19005
- WO-A-2006/121887
- WO-A1-2004/008997
- US-A- 4 755 593
- US-A1- 2003 225 355

## Description

### Introduction

This invention relates to a tissue repair implant.

US patent application published under number US 2003/225355 describes a composite material for wound repair comprising a biodegradable barrier material and a support material.

### Statements of Invention

According to the invention there is provided a tissue repair implant comprising a layer of peritoneal membrane and a support member to reinforce the layer of peritoneal membrane.

The layer of peritoneal membrane acts to prevent adhesion of internal organs or tissue structures and acts as a native peritoneum remodelling template. By providing the support member, this arrangement increases the mechanical strength of the implant.

In one embodiment of the invention the support member comprises a biological material. The support member may comprise a non-peritoneal biological material.

In another embodiment the support member comprises a non-biological material. The support member may comprise a synthetic material. The synthetic material support member provides permanent reinforcement while the peritoneal membrane supports the native tissue remodelling over time.

The support member may comprise a biodegradable material. The support member may comprise a non-biodegradable material.

In one case the implant comprises means to attach the layer of peritoneal membrane to the support member. The implant may comprise a laminate of the layer of peritoneal membrane and the support member. The support member may be at least partially encapsulated in the peritoneal membrane. The implant may comprise a first layer of peritoneal membrane and a second layer of peritoneal membrane. The support member may be located between the first layer of peritoneal membrane and the second layer of peritoneal membrane. The support member may comprise one or more openings extending at least partially therethrough. The support member may comprise a mesh. The layer of peritoneal membrane may extend at least partially through the opening of the support member to attach the layer of peritoneal membrane to the support member. The support member may comprise means to grip the layer of peritoneal membrane extending at least partially through the opening.
The attachment means may comprise a suture element, and/or a staple element. The attachment means may comprise an adhesive bond. The layer of peritoneal membrane may be lyophilised to the support member.

In another case the layer of peritoneal membrane is configured to be remodelled by in-growth of tissue. The layer of peritoneal membrane may comprise means to promote in-growth of tissue. The layer of peritoneal membrane may promote in-growth of tissue through the nature of the peritoneal membrane and through a plurality of pores. The layer of peritoneal membrane may comprise a plurality of pores.

In one embodiment the layer of peritoneal membrane and/or the support member is configured to be attached to a tissue wall. The layer of peritoneal membrane and/or the support member may be configured to be attached to an interior wall of an abdominal tissue. The implant may comprise means to attach the layer of peritoneal membrane and/or the support member to a tissue wall. The implant may allow attachment of the layer of peritoneal membrane and/or the support member to a tissue wall. The attachment means may comprise a suture element, and/or a staple element.

In another embodiment the implant is configured to treat an abdominal hernia repair, and/or a pelvic floor reinforcement, and/or a vaginal reconstruction, and/or a uterine wound cover, and/or a colorectal application, and/or a urological application, and/or a gynaecological application. For some applications the strength requirement of the implant may be greater such as for prolapse repair. The shape of the implant may be pre-defined to suit the shape of an abdominal cavity. The shape of the implant may be pre-defined to suit the shape of an area of application.

In another case the implant is movable between a delivery configuration and a deployment configuration. The implant may be substantially collapsed in the delivery configuration. The implant may be substantially expanded in the deployment configuration. The implant may comprise means to bias the implant towards the deployment configuration. The biasing means may comprise a biodegradable material.

In one embodiment the layer of peritoneal membrane is derived from a segment of an abdominal wall of a warm blooded vertebrate. The layer of peritoneal membrane may be delaminated from the abdominal wall of the warm blooded vertebrate. The layer of peritoneal membrane may comprise an allograft membrane derived from a human donor. The layer of peritoneal membrane may comprise a xenograft membrane derived from an animal. The layer of peritoneal membrane may comprise a xenograft membrane derived from a bovine or porcine source. The porcine peritoneal membrane may be derived from a sow population of screened donors. The layer of peritoneal membrane may comprise a bovine or porcine membrane. The layer of peritoneal membrane may comprise a porcine membrane derived from a sow. The layer of peritoneal membrane may be decellularised. The layer of peritoneal membrane may be lyophilised.

In another aspect of the invention there is provided a tissue implant for treating abdomino-pelvic defects, said tissue implant comprising: a support member for engaging, attaching, and remodelling with an inside and/or organ contacting surface of an abdomino-pelvic wall; and a layer of peritoneal membrane attached to said support member, said layer of peritoneal membrane covering the support member to prevent the formation of adhesions between the support member and one or more organs.

The invention also provides in a further aspect an apparatus for grafting and/or repairing an abdominal wall, the abdominal wall having an inside surface that defines a cavity for containing the internal organs, said apparatus comprising: a prosthetic support and/or reinforcement member having inner and outer surfaces, said outer surface for engaging and adhering to the inside surface of the abdominal wall: and a layer of biologic tissue being attached to said inner surface of said prosthetic reinforcement, said layer of biologic tissue extending over a portion of the inwardly facing surface of the prosthetic reinforcement to provide resistance to adhesion formation and internal organ attachment as wound healing and tissue ingrowth into the prosthetic reinforcement occurs; said layer of biologic tissue being selected from a group consisting of peritoneal tissue.

According to the invention there is provided a tissue repair implant comprising a membrane of peritoneal tissue.

The implant assists in tissue repair by providing additional mechanical strength to the abdominal wall. For example in the case of a wound opening by covering the wound opening with the implant. The in-growth of surrounding abdominal wall tissue into the implant further improves the wound healing.

The implant will come into contact with the bowel and internal organs. The peritoneal tissue acts to minimise or discourage organ adhesion to the implant.

In one embodiment of the invention the membrane of peritoneal tissue is configured to be attached to an interior wall of an abdominal tissue. Preferably the implant comprises means to attach the membrane of peritoneal tissue to an interior wall of an abdominal tissue. The attachment means may comprise a suture element, and/or a staple element. Most preferably the implant is configured to treat an abdominal hernia repair, and/or a pelvic floor reinforcement, and/or a vaginal reconstruction, and/or a uterine wound cover, and/or a colorectal application, and/or a urological application, and/or a gynecological application.

In one case the shape of the implant is pre-defined to suit the shape of an abdominal cavity.

In another embodiment the membrane of peritoneal tissue is derived from a segment of an abdominal wall of a warm blooded vertebrate. Preferably the membrane of peritoneal tissue is delaminated from the abdominal wall of the warm blooded vertebrate. The membrane of peritoneal tissue may comprise an allograft membrane derived from a human donor. The membrane of peritoneal tissue may comprise a xenograft membrane derived from an animal. Preferably the membrane of peritoneal tissue comprises a bovine or porcine membrane.

In another case the membrane of peritoneal tissue is sterilised. Preferably the membrane of peritoneal tissue is lyophilised.

In one embodiment the implant comprises a support member to reinforce the membrane of peritoneal tissue. In the case where there is large strength requirement, for example in the case of a large opening during hernia repair, the support member provides additional strength. In the case where there is less or no load bearing, for example in colorectal or women's health applications, the support member may not be required. Preferably the support member comprises a mesh. The support member may comprise a non-biological material. The support member may comprise a synthetic material. The support member may comprise a biodegradable material.

In one case the implant comprises means to attach the membrane of peritoneal tissue to the support member. Preferably the support member is at least partially encapsulated in the membrane of peritoneal tissue. The implant may comprise a first membrane layer of peritoneal tissue and a second membrane layer of peritoneal tissue. Most preferably the support member is located between the first membrane layer of peritoneal tissue and the second membrane layer of peritoneal tissue.

In another embodiment the support member comprises at least one opening extending at least partially therethrough. Preferably the membrane of peritoneal tissue extends at least partially through the opening of the support member to attach the membrane of peritoneal tissue to the support member. The support member may comprise means to grip the membrane of peritoneal tissue extending at least partially through the opening.

In another case the attachment means comprises a suture element, and/or a staple element. The membrane of peritoneal tissue may be bonded to the support member. The membrane of peritoneal tissue may be lyophilised to the support member. The membrane of peritoneal tissue may be attached to the support member by means of electrospinning.

In one embodiment the implant is configured to carry one or more biologically active agents. Preferably the membrane of peritoneal tissue is configured to carry one or more biologically active agents.

In one case the implant is movable between a delivery configuration and a deployment configuration. Preferably the implant is substantially collapsed in the delivery configuration. The implant may be substantially expanded in the deployment configuration. Most preferably the implant comprises means to bias the implant towards the deployment configuration. The biasing means may comprise a biodegradable material.

In another aspect of the invention there is also provided a method of preparing a tissue repair implant of the invention.

In one embodiment of the invention the method comprises the step of shaping the implant to suit the shape of an abdominal cavity.

In one case the method comprises the step of deriving the membrane of peritoneal tissue from a segment of an abdominal wall of a warm blooded vertebrate.

In another embodiment the method comprises the step of sterilising the membrane of peritoneal tissue.

In another case the method comprises the step of attaching the membrane of peritoneal tissue to a support member. Preferably the method comprises the step of at least partially extending the membrane of peritoneal tissue through an opening of the support member. The membrane of peritoneal tissue may be drawn through the opening of the support member. The membrane of peritoneal tissue may be pushed through the opening of the support member. The membrane of peritoneal tissue may be extended through the opening of the support member by application of a pressure differential. The membrane of peritoneal tissue may be extended through the opening of the support member by application of a mechanical force.

In one embodiment the method comprises the step of at least partially encapsulating the support member in the membrane of peritoneal tissue. The membrane of peritoneal tissue may be bonded to the support member. The membrane of peritoneal tissue may be lyophilised to the support member. The membrane of peritoneal tissue may be attached to the support member by means of electrospinning.

The invention also provides in a further aspect a method of repairing tissue, the method comprising the step of attaching a membrane of peritoneal tissue to an interior wall of the tissue.

In one embodiment of the invention the method comprises a method of repairing an abdominal tissue. The method may comprise a method of treating an abdominal hernia repair, and/or a pelvic floor reinforcement, and/or a vaginal reconstruction, and/or a uterine wound cover, and/or a colorectal application, and/or a urological application, and/or a gynecological application.

In one case the method comprises the step of delivering the membrane of peritoneal tissue to a deployment site at the interior wall of the tissue. Preferably the membrane of peritoneal tissue is delivered laparoscopically. Ideally the method comprises the step of collapsing the membrane of peritoneal tissue to a delivery configuration prior to the step of delivery. Most preferably the method comprises the step of causing expansion of the membrane of peritoneal tissue to a deployment configuration after the step of delivery.

According to a further aspect of the invention there is provided an apparatus for (grafting) repairing the abdominal wall, the abdominal wall having an inside surface that defines a cavity for containing the internal organs, said apparatus comprising: a prosthetic (support) reinforcement member having inner and outer surfaces, said outer surface for engaging and adhering to the inside surface of the abdominal wall: and a layer of biologic tissue being attached to said inner surface of said prosthetic reinforcement, said layer of biologic tissue extending over a portion of the inwardly facing surface of the prosthetic reinforcement to provide resistance to adhesion formation and internal organ attachment as wound healing and tissue ingrowth into the prosthetic reinforcement occurs; said layer of biologic tissue being selected from a group consisting of peritoneal tissue.

The invention also provides in another aspect a tissue graft comprising the peritoneal membrane of a segment of the abdominal wall of a warm blooded vertebrate, said peritoneal membrane being delaminated from the abdominal wall.

According to a further aspect of the invention there is provided a tissue graft comprising two or more layers of peritoneal membrane of a segment of the abdominal wall of a warm blooded vertebrate, said peritoneal membrane being delaminated from the abdominal wall.

The invention also provides in another aspect a collagenous biomaterial medical device, comprising: a laminate having a plurality of layers of (lyophilized) peritoneal membrane.

According to a further aspect of the invention there is provided a graft prosthesis comprising: a purified, collagen based matrix removed from a peritoneal membrane source, said purified structure having a contaminant level making said purified structure biocompatible, said purified structure having a low endotoxin level.

The use of the biologic peritoneal membrane in combination with other materials offers a longer term wound covering solution and tissue repair that enhances the wound healing response while preventing adhesion formation.

The biologic membrane as a composite implant, which consists of a synthetic mesh encapsulated within a biologic membrane component, provides a means of adhesion prevention, reinforcement, and augmentation which satisfies the need for tissue regeneration through hosting tissue remodelling, and displays comparable mechanical properties to the adjacent normal tissue, and prevents adhesion formation during host tissue remodelling.

The placement of the peritoneal membrane tissue of allo- or xeno-derived source over the synthetic mesh, at the location of reinforcement/repair leads to superior integration of the synthetic mesh within the surrounding tissue, which naturally leads to minimal/loss of fibrous encapsulation, which in turn leads to lower synthetic mesh shrinkage; and presentation of an adhesion barrier leading to minimal/lack of associated intestinal adhesions, eviscerations or chronic pain.

The peritoneal membrane is processed to remove any immunogenic material, such as cells, viruses, and undergoes appropriate sterilization. The invention provides a processed and sterile peritoneal membrane from either allo- or xeno-source for wound covering, and also provides a processed and sterile peritoneal membrane from either allo- or xeno-source for reinforcement and/or repair.

### Brief Description of the Drawings

The invention will be more clearly understood from the following description of some embodiments thereof, given by way of example only, with reference to the accompanying drawings, in which:
Fig. 1 is an isometric view of a tissue repair implant according to the invention with a peritoneal membrane pocket with a surgical mesh material inserted into the pocket, the composite material may be sealed through peripheral stitching, with no peritoneal membrane perforations, or with peritoneal membrane perforations,
Fig. 2 is a side view of another tissue repair implant according to the invention with suturing of a membrane to a mesh,
Fig. 3 is a photographic image illustrating assembly of another tissue repair implant according to the invention with a suture attachment method,
Fig. 4 is an exploded isometric view of another tissue repair implant according to the invention with a lyophilisation method,
Fig. 5 is a side view of the implant of Fig. 4 assembled,
Fig. 6 is an exploded isometric view illustrating assembly of another tissue repair implant according to the invention with a lyophilisation method,
Fig. 7 is an exploded isometric view illustrating assembly of another tissue repair implant according to the invention with a lyophilisation method,
Fig. 8 is a plan view of another tissue repair implant according to the invention with a peripheral tab attachment,
Fig. 9 is an isometric view of another tissue repair implant according to the invention which may be applied as a uterine wound covering,
Fig. 10 outlines the tensile properties between processed human Fascia Lata and processed human peritoneal membrane in both the X and Y directions,
Fig. 11 shows the differences in useable surface area of peritoneal membrane between different populations of pigs,
Fig. 12 shows the average burst strength of porcine derived peritoneal membrane between different populations,
Fig. 13 shows the cellular presence and structural retention of processed porcine peritoneal membranes derived from various populations,
Fig. 14 shows the mechanical difference between human and porcine peritoneal membrane,
Fig. 15 shows the extent of adhesions and tissue integration between a MotifMesh and a MotifMesh with a layer of human peritoneal membrane in a rodent implant study, and
Fig. 16 shows the surface area of adhesions found between a MotifMesh and a MotifMesh with a layer of human peritoneal membrane in a rodent implant study.

### Detailed Description

In this patent specification the term "material" includes any synthetic, biologically derived or bioactive component which may be single- or multi-layered; "composite material" represents any combination of synthetic, biologically derived or bioactive components. In this patent specification the term "regeneration" is understood as the process of anatomical and physiological restoration of a tissue to its normal structure and function. In this patent specification the term "bioactive" is defined as either a biological tissue of human (allograft) or xenograft source that supports the process of wound healing leading to tissue regeneration.

A peritoneal tissue derived from a segment of an abdominal wall of a warm blooded vertebrate may comprise a peritoneal membrane, muscle, adipose tissue, and two fascia layers. The peritoneal membrane is thus one of a number of constituent parts of a peritoneal tissue.

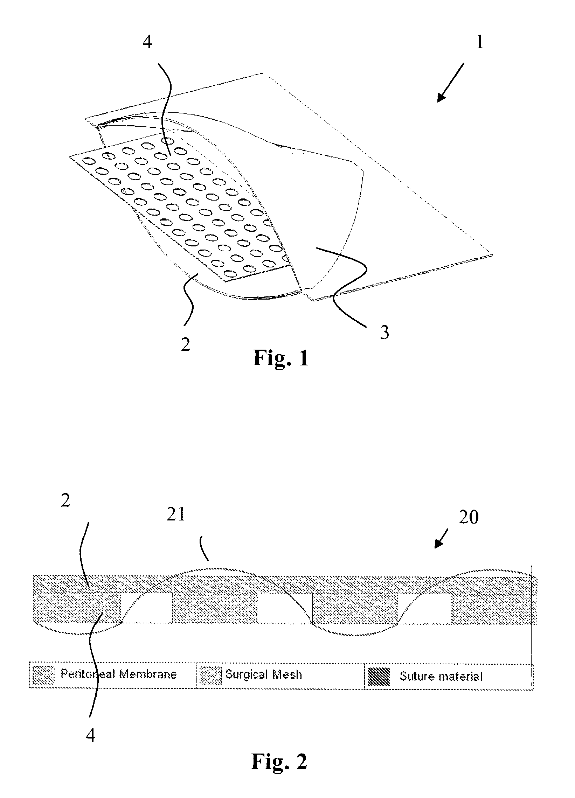

Referring to the drawings, and initially to Fig. 1 thereof, there is illustrated a tissue repair implant 1 according to the invention. The implant 1 may be employed to treat an abdominal hernia repair, and/or a pelvic floor reinforcement, and/or a vaginal reconstruction, and/or a uterine wound cover, and/or a colorectal application, and/or a urological application, and/or a gynaecological application.

The implant 1 comprises a lower layer of peritoneal membrane 2, an upper layer of peritoneal membrane 3, and a support member 4 to reinforce the two layers of peritoneal membrane 2, 3.

The implant 1 comprises a laminate of the lower layer of peritoneal membrane 2, the upper layer of peritoneal membrane 3, and the support member 4. The support member 4 is located between the lower layer of peritoneal membrane 2 and the upper layer of peritoneal membrane 3. The support member 4 is encapsulated between the two layers of peritoneal membrane 2, 3 to attach the two layers of peritoneal membrane 2, 3 to the support member 4.

In this case the support member 4 comprises a non-biological synthetic material mesh. The support member 4 comprises a plurality of openings extending therethrough.

The support member 4 may alternatively comprise a biological material, such as a non-peritoneal biological material.

The support member 4 may comprise a biodegradable material. Alternatively the support member 4 may comprise a non-biodegradable material.

The implant 1 is movable between a collapsed delivery configuration and an expanded deployment configuration. The implant 1 may comprise means to bias the implant 1 towards the deployment configuration. The biasing means may comprise a biodegradable material.

Either of the layers of peritoneal membrane 2, 3 and/or the support member 4 may be attached to an interior wall of an abdominal tissue by means of a suture element, and/or a staple element. Either of the layers of peritoneal membrane 2, 3 may be remodelled by in-growth of tissue. Either of the layers of peritoneal membrane 2, 3 may comprise a plurality of pores to promote in-growth of tissue.

In another embodiment, a synthetic/biological adhesive may be used to bond the peritoneal membranes 2, 3 to the support member 4.

This embodiment may use an adhesive to bond the membranes 2, 3 to the mesh/scaffold layer/scaffold 4. In order to attach the biological membranes 2, 3 to the mesh/scaffold layer/scaffold 4, a biologic adhesive for example glutaraldehyde based, or a synthetic adhesive may be used.

Each layer of peritoneal membrane 2, 3 is derived from a segment of an abdominal wall of a warm blooded vertebrate, and is delaminated from the abdominal wall of the warm blooded vertebrate. Either of the layers of peritoneal membrane 2, 3 may comprise an allograft membrane derived from a human donor, or may comprise a xenograft membrane derived from an animal, such as a bovine or porcine membrane. Each layer of peritoneal membrane 2, 3 is decellularised and is lyophilised.

The peritoneal tissue derived from the segment of the abdominal wall of the warm blooded vertebrate comprises the peritoneal membrane, muscle, adipose tissue, and two fascia layers.

The peritoneal tissue is processed after harvesting from the abdominal wall of the warm blooded vertebrate. The processing includes removing the muscle and adipose tissue from the peritoneal tissue, screening the peritoneal tissue for viruses such as Hepatitis B, and cleaning the peritoneal tissue to remove viruses, bacteria, and the adipose tissue.

After the processing of the peritoneal tissue, the two fascia layers merge into a single fascia layer. The single fascia layer enhances the mechanical strength of the peritoneal membrane in both axes.

After the processing of the peritoneal tissue, the collagen content in all layers of the peritoneal tissue is higher and the toxin level in all layers of the peritoneal tissue is lower compared with the peritoneal tissue before processing.

In use, the implant 1 is collapsed to the delivery configuration and inserted into the abdominal/pelvic cavity of a patient, for example through a laparoscopic incision. The implant 1 is advanced to a desired treatment site in the abdominal/pelvic cavity, for example by means of a delivery catheter. When the implant 1 reaches the desired treatment site, the implant 1 is expanded to the deployment configuration.

Either of the layers of peritoneal membrane 2, 3 and/or the support member 4 is attached to an interior wall of an abdominal tissue by means of the suture element, and/or the staple element. The implant 1 may thus be employed to treat an abdominal hernia repair, and/or a pelvic floor reinforcement, and/or a vaginal reconstruction, and/or a uterine wound cover, and/or a colorectal application, and/or a urological application, and/or a gynaecological application.

Over time one or more of the layers of peritoneal membrane 2, 3 is remodelled by in-growth of tissue.

Fig. 2 illustrates a further tissue repair implant 20 according to the invention, which is similar to the tissue repair implant 10 of Fig. 1, and similar elements in Fig. 2 are assigned the same reference numerals.

In this case the implant 20 comprises a suture element 21 to attach the layer of peritoneal membrane 2 to the support member 4.

Mechanical attachment is achieved using suturing or stapling. Attachment of the membrane 2 is achieved through the use of a suturing or stapling method whereby the inherent porosity of the mesh 4 is used as an attachment point while the membrane 2 may have an inherent porosity suited to a specific suturing/stapling pattern. In order to attach the membrane 2 to the scaffold 4, pre-determined apertures are required for suturing. Alternatively pattern puncturing at key scaffold locations may be used to attach the membrane 2 onto the scaffold 4. Another alternative may be to staple the membrane 2 to the underlying mesh/scaffold 4 in a pattern suitable with the mesh porosity without compromising its inherent mechanical properties, as illustrated in Fig. 2.

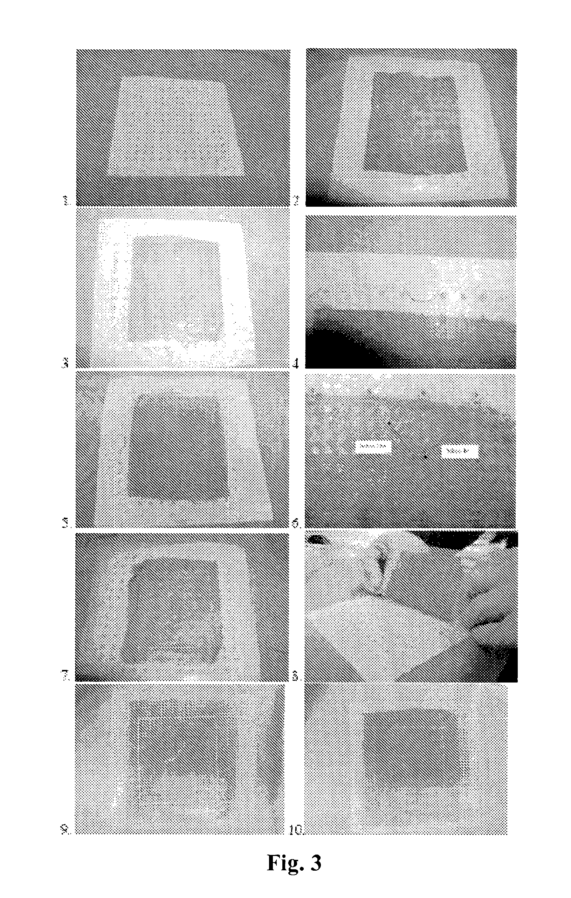

Suture attachment is used in the case of Fig. 3. This method involves the use of a running suture to attach the membrane 2 to the synthetic mesh 4 along the perimeter of the synthetic mesh 4. In addition, a single point suture was used to attach the membrane 2 at the centre of the synthetic mesh 4. A suture guide was provided which allowed for easy and consistent line-up of the synthetic mesh 4 and the membrane 2 when suturing. This was achieved through the following steps:

Step 1 involved the placing the suture guide on a sterile surface. Step 2 involved placing the membrane 2 onto the suture guide. Step 3 involved placing the synthetic mesh 4 over the membrane 2. Step 4 involved inserting the synthetic mesh 4 onto the tabs provided on the suture guide. Step 5 shows the suture guide-membrane 2-synthetic mesh 4 complex. Step 6 involves the suturing of the membrane 2 to the synthetic mesh 4 via the allotted holes located at the major strut intersection of the synthetic mesh 4. Step 7 shows the perimeter running suture. Step 8 involves the peeling back of the membrane 2-synthetic mesh 4 complex. Steps 9 and 10 show the complex on both sides. These steps are illustrated in Fig. 3. This method revealed a minimum peel force of approximately 3.1N.

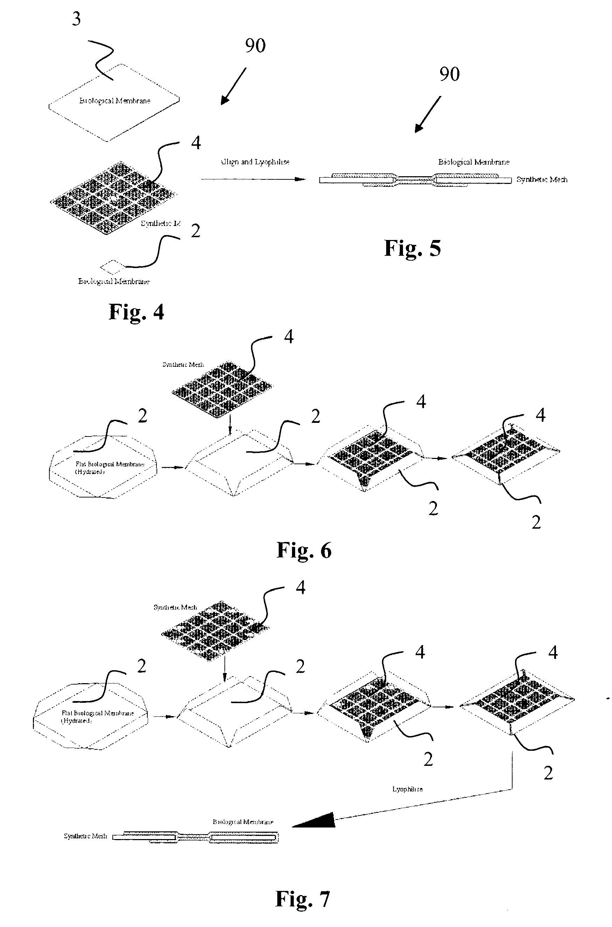

Referring to Figs. 4 and 5 there is illustrated another tissue repair implant 90 according to the invention, which is similar to the tissue repair implant 1 of Fig. 1, and similar elements in Figs. 4 and 5 are assigned the same reference numerals.

In this case the layers of peritoneal membrane 2, 3 are lyophilised to the support member synthetic mesh 4.

This method involves the use of freeze-drying of the membranes 2, 3 onto the synthetic mesh 4. Lyophilisation of the biologic membranes 2, 3 on both sides of the synthetic mesh 4 at specific points allows for the connection of the two membrane components 2, 3 through the pores of the synthetic mesh 4 or through an alteration in the mesh design for example designated larger pores. In order for this to occur successfully, the two layers of biological membrane 2, 3 may need to be pressed together so vacuum packing may be required, as illustrated in Figs. 4 and 5.

The membrane 2 may be lyophilised in a folder pattern at the periphery so that the synthetic mesh 4 may be placed into the membrane 2 like an envelope. Once lyophilised, the membrane 2 would be rigid around the folder sections and hence keep the synthetic mesh 4 within it, as illustrated in Fig. 6.

Another method of lyophilisation involves using the two previously described concepts of Figs. 4 and 5 and Fig. 6, whereby the second method is applied but is also pressed together allowing for the attachment of the two pieces of biological membrane 2, 3 to bond together at the periphery. This bonding may be achieved through the native pores of the synthetic mesh 4 or through alterations of the mesh 4, for example designated larger pores, as illustrated in Fig. 7.

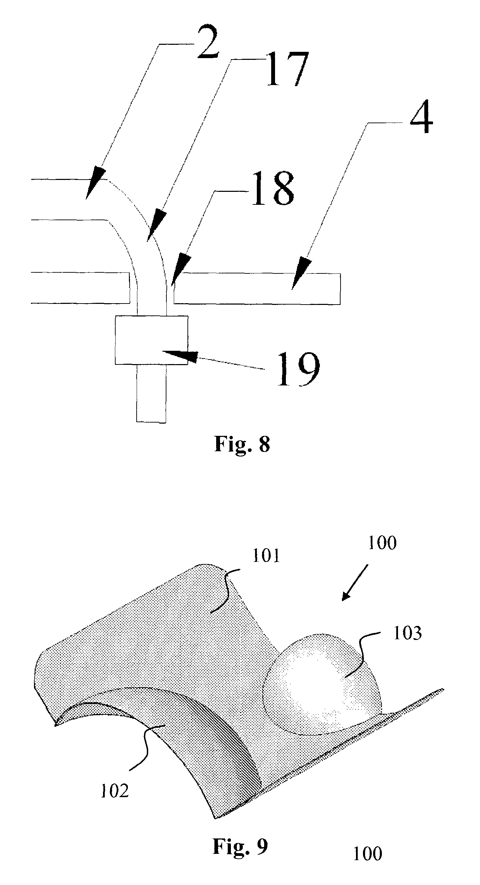

Membrane tabbing is used in the case of Fig. 8. This method involves the use of tabs 17 arising from the peritoneal membrane 2 to loop through one or more slits 18 in the synthetic mesh 4 and therefore attach it. A clamp 19 placed on the membrane strand distal to the mesh insertion will fix it to the synthetic mesh 4, as illustrated in Fig. 8.

In Fig. 9 there is illustrated another tissue repair implant 100 according to the invention, which is similar to the tissue repair implant 1 of Fig. 1, and similar elements in Fig. 9 are assigned the same reference numerals. The implant 100 is preformed to meet a specific area of application.

In this case the shape of the implant 100 is pre-defined to suit the shape of a hysterectomised pelvic floor. The implant 100 has an outlying profile 101 to meet the curve of the pelvic floor as well as protruding surfaces to conform to the rectum 103 and bladder 102.

It will be appreciated that the one or more layers of peritoneal membrane may be attached to the support member by variety of possible means of attachment, such as:
1. A staple element.
2. Attachment of the membrane to the mesh/scaffold through the use of an inherently adhesive material
   In order to achieve an adequate bond, an adhesive membrane may be used. Encapsulation by a synthetic membrane may be achieved through the use of a material with a low glass transition temperature (T_{g}) - which may be lower than normal operating room temperature. In the case of a biological membrane, the encapsulation may take advantage of alginates, which may be in a lyophilized form, to impart an adequate amount of adhesive properties.
3. Sewing attachment

Rather than manually suturing the two materials together, sewing may be a more time efficient method. A predetermined pattern may be imparted into the synthetic mesh which would accommodate the sewing pattern. Non-portable sewing machines are available for medical purposes and have been used extensively in cardiovascular implants, namely Abdominal Aortic Aneurysm stent fabrication.

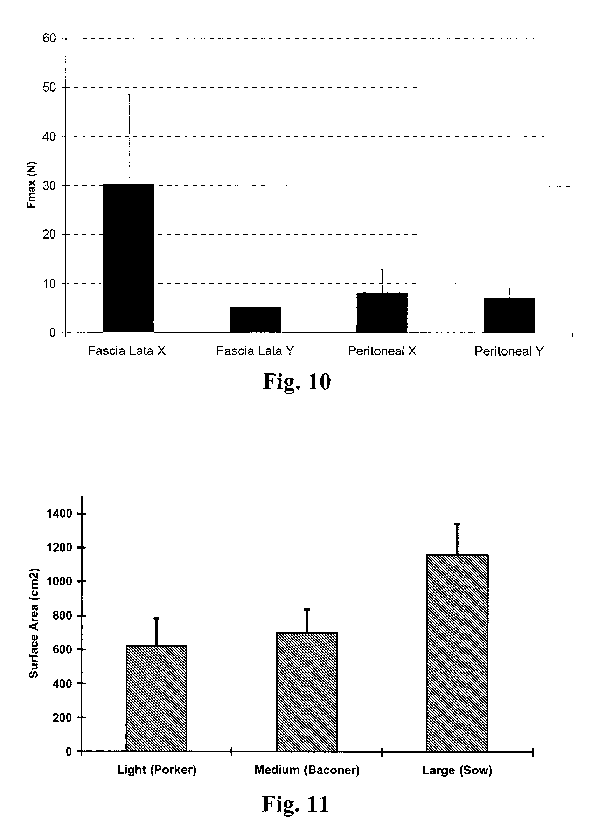

Fig. 10 shows the tensile testing results between peritoneal membrane and Fascia Lata membranes in both the X and Y directions.
Fig. 11 shows the difference in useable surface area of peritoneal membrane among various porcine populations.

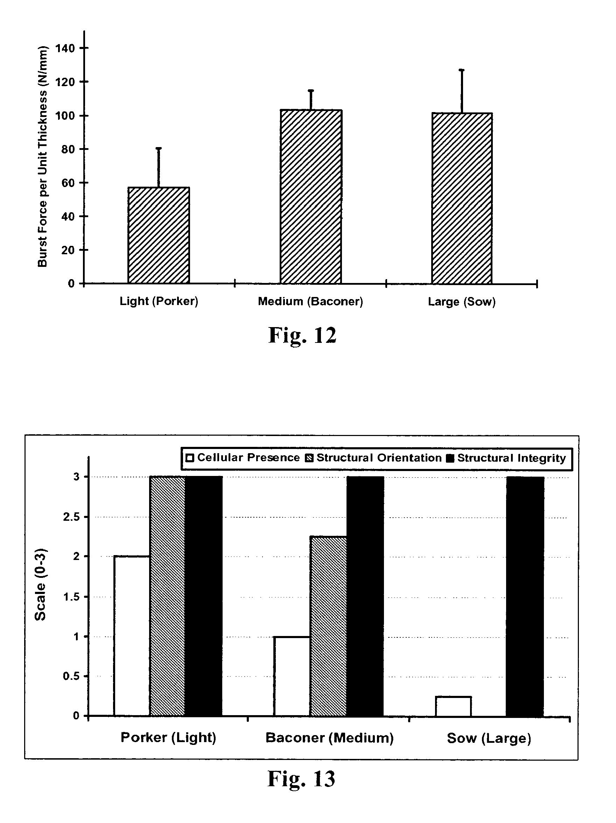

Fig. 12 shows the difference in mechanical properties of peritoneal membrane among various porcine populations.
Fig. 13 shows the difference in cellular presence and structural retention of processed peritoneal membrane among various porcine populations.

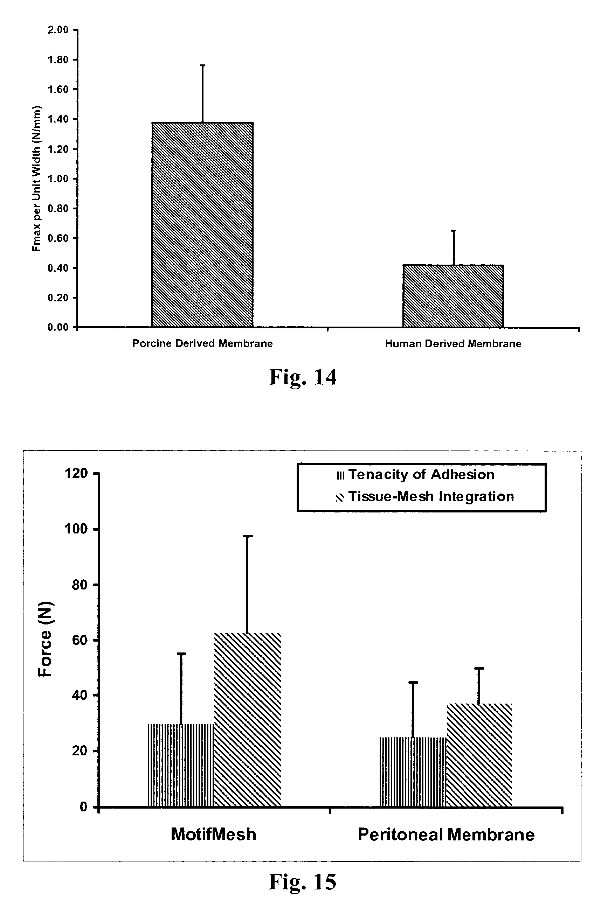

Fig. 14 shows the difference between the mechanical properties of porcine derived and human derived peritoneal membranes.
Fig. 15 shows the difference in the adhesion formation and tissue integration of a MotifMesh (cPTFE) with and without human peritoneal membrane encapsulated around it. Results are from a rodent implant study.

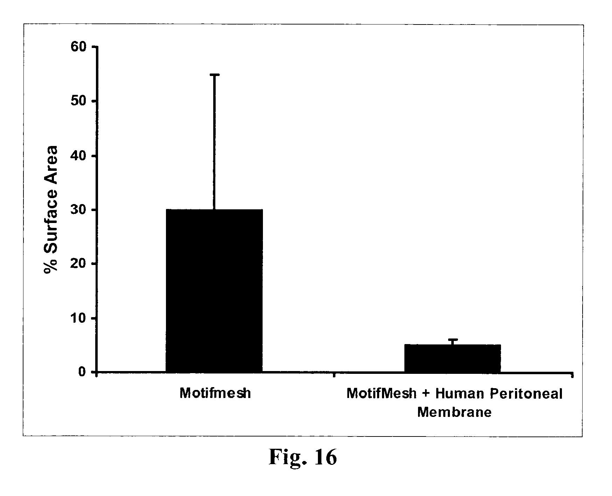

Fig. 16 shows the difference in the percentage surface area of adhesions between a MotifMesh (cPTFE) with and without human peritoneal membrane encapsulated around it. Results are from a rodent implant study.

According to the invention a composite material has been developed for regenerating, reinforcing, or augmenting soft tissue defects in a patient. Such an approach combines the predictable and reliable mechanical properties of a synthetic mesh with the regenerative characteristics of mechanically weaker, yet biologically active materials. The composite implant may consist of a synthetic material composed of one or multiple layers attached or encapsulated into a bioactive and flexible material construct. The encapsulating bioactive material may be micro or macro patterned to allow physiological fluid transport and enhance tissue incorporation, depending on the specific application.

According to the invention a biologic membrane material has been developed to meet criteria for wound covering, tissue reinforcement, and augmentation. In particular, a biologic membrane with adequate mechanical support and the ability to stimulate tissue regeneration are features of the membrane. The present invention addresses the shortcomings of current reinforcement and augmentation solutions related to general, ob/gyn, and colo-rectal surgery. The biologic membrane may be peritoneal membrane from an allograft or xenograft source. In another embodiment a synthetic surgical mesh is combined with the biologic membrane through mechanical means. In another embodiment, a synthetic surgical mesh is encapsulated by the biologic membrane. Apertures may be created within the composite material to allow for fluid transport. The biologic membrane may be incorporated with bioactive agents, such as growth factors, antibiotics or other wound healing/tissue regeneration enhancing factors, through physical or chemical means of attachment. In yet another embodiment, a biologic tissue matrix layer is attached for example mechanically, or chemically, or thermally, on one side of a synthetic surgical mesh to selectively encourage tissue integration/regeneration and the biologic membrane is attached to the other side to improve the wound healing response on the surface exposed to the intra-abdominal organs and contents to act as an anti-adhesive barrier.

### Example 1: Determination of a suitable membrane for adhesion prevention

Two membranes were chosen as potential adhesion preventing membranes. Both peritoneal membrane and fascia lata were chosen due to their natural anti-adhesion anatomy. The mechanical properties of each membrane were investigated. It was found that peritoneal membrane behaves more isotropic than fascia lata, hence proving more appropriate as a soft tissue implant. Fig. 10 illustrates the tensile testing results.

### Example 2:

The characterisation of xenograft peritoneal membrane procured from three different porcine populations: Porker, Baconer and Sow were investigated. Membranes from various populations of pigs were analysed for useable surface area and burst strength. It was found that the large (sow derived) membrane offers the largest workable surface area, and the medium (baconer derived) and large (sow derived) membranes offer the highest burst strength in comparison to light (porker derived) membranes.
Fig. 11 illustrates the surface area results. Fig. 12 illustrates the mechanical analysis results.

### Example 3: Synthetic mesh encapsulated by a peritoneal membrane for hernia repair

The peritoneal membrane is harvested and processed to create a decellularized tissue, which is then lyophilized. The lyophilized peritoneal membrane is cut to shape or folded to accommodate the size of a surgical mesh such as a surgical mesh available under the tradename Motifmesh^{™}. The peritoneal membrane is fitted over the surgical mesh using continuous suture stitching, lyophilization, or biological glues such as fibrin, cyanoacrylates, or any other method that those versed in the art may use to allow handling of the composite material without mesh to peritoneal membrane separation during the placement procedure.

In the case of a composite surgical mesh for soft tissue repair, methods of attachment of allograft peritoneal membrane onto a PTFE mesh, such as that available under the tradename MotifMesh, were investigated. The various options available in achieving an adequate method for attachment of a biological membrane to a synthetic mesh were investigated. It was found that based on experimentation, the most practical method of attaching a membrane to a synthetic mesh involves suturing/sewing or stitching the various parts together.

### Example 4: Pre-Clinical performance of a synthetic mesh encapsulated by a peritoneal membrane in a rodent hernia model

The performance of the composite outlined in Example 3 (suture stitching) in a chronic hernia repair rodent model was evaluated. It was found that the use of the peritoneal membrane in combination with a MotifMesh^{™} (cPTFE) led to the least extent and tenacity of adhesions. It was found that the peritoneal membrane handled well in its desired hydrated form. Fig. 15 illustrates the mechanical characterisation.
Fig. 16 illustrates the percentage surface area of adhesions.

### Example 5:

The in vivo performance of a synthetic mesh encapsulated by a peritoneal membrane was evaluated in a large hernia animal model. Free range pigs were used in an intra-abdominal placement of the implant. In a swine model the peritoneal membrane dual construct was anchored onto the abdominal wall. It was found that a MotifMesh^{™} alone displayed a more robust host tissue response than that of a peritoneal membrane dual construct comprising a peritoneal membrane encapsulated MotifMesh^{™}, illustrated by less mature collagen presence and enhanced inflammatory cellularity at location. Thus the peritoneal membrane dual construct provides a more conducive healing environment than MotifMesh^{™} alone.

Possible applications for the tissue repair implant of the invention include laparotomy closure/abdominal wall; in general surgery: ventral intraperitoneal hernia repair, or staple line reinforcement; in colo-rectal: abdominoperineal excision of the rectum, or ostomy creation and closure; in urology/gynecology: abdominal/pelvic pain secondary to adhesions, or hysterectomy, myomectomy, and ovariectomy.

The tissue repair implant of the invention reduces or prevents adhesion formation. The peritoneal membrane provides the function of reinforcement and augmentation as a biological scaffold for clinical applications.

The invention is not limited to the embodiments hereinbefore described, with reference to the accompanying drawings, which may be varied in construction and detail.

## Claims

1. A tissue repair implant comprising a layer of peritoneal membrane and a support member to reinforce the layer of peritoneal membrane, the layer of peritoneal membrane being derived from a segment of an abdominal wall of a warm blooded vertebrate, the layer of peritoneal membrane comprising a xenograft membrane derived from an animal, the layer of peritoneal membrane comprising a xenograft membrane derived from a porcine source, the layer of peritoneal membrane comprising a porcine membrane derived from a sow, the implant being movable between a delivery configuration and a deployment configuration, the implant comprising means to bias the implant towards the deployment configuration, the biasing means comprising a biodegradable material.

2. An implant as claimed in claim 1 wherein the support member comprises a biological material.

3. An implant as claimed in claim 1 or 2 wherein the implant comprises means to attach the layer of peritoneal membrane to the support member.

4. An implant as claimed in any of claims 1 to 3 wherein the implant comprises a laminate of the layer of peritoneal membrane and the support member.

5. An implant as claimed in claim 3 or 4 wherein the support member is at least partially encapsulated in the peritoneal membrane.

6. An implant as claimed in any of claims 1 to 5 wherein the implant comprises a first layer of peritoneal membrane and a second layer of peritoneal membrane.

7. An implant as claimed in claim 6 wherein the support member is located between the first layer of peritoneal membrane and the second layer of peritoneal membrane.

8. An implant as claimed in any of claims 1 to 7 wherein the support member comprises one or more openings extending at least partially therethrough.

9. An implant as claimed in claim 8 wherein the layer of peritoneal membrane extends at least partially through the opening of the support member to attach the layer of peritoneal membrane to the support member.

10. An implant as claimed in claim 9 wherein the support member comprises means to grip the layer of peritoneal membrane extending at least partially through the opening.

11. An implant as claimed in any of claims 1 to 10 wherein the layer of peritoneal membrane comprises means to promote in-growth of tissue.

12. An implant as claimed in any of claims 1 to 11 wherein the shape of the implant is pre-defined to suit the shape of an abdominal cavity.

## Patentansprüche

1. Gewebereparaturimplantat, das eine Schicht von peritonealer Membran und ein Stützelement zur Verstärkung der Schicht von peritonealer Membran umfasst, wobei die Schicht von peritonealer Membran von einem Segment einer Bauchdecke eines warmblütigen Wirbeltiers stammt, die Schicht von peritonealer Membran eine Xenotransplantat-Membran umfasst, die von einem Tier stammt, die Schicht von peritonealer Membran eine Xenotransplantat-Membran umfasst, die von einem Schwein stammt, die Schicht von peritonealer Membran eine Schweinemembran umfasst, die von einer Sau stammt, das Implantat zwischen einer Konfiguration der Zufiihrung und einer Konfiguration der Anordnung beweglich ist, das Implantat Mittel umfasst, um das Implantat zur Konfiguration der Anordnung auszurichten, wobei das Ausrichtungsmittel ein biologisch abbaubares Material umfasst.

2. Implantat nach Anspruch 1, worin das Stützelement ein biologisches Material umfasst.

3. Implantat nach Anspruch 1 oder 2, worin das Implantat Mittel zur Verbindung der Schicht von peritonealer Membran mit dem Stützelement umfasst.

4. Implantat nach einem der Ansprüche 1 bis 3, worin das Implantat einen Verbund der Schicht von peritonealer Membran und des Stützelements umfasst.

5. Implantat nach Anspruch 3 oder 4, worin das Stützelement zumindest teilweise in der peritonealen Membran eingeschlossen ist.

6. Implantat nach einem der Ansprüche 1 bis 5, worin das Implantat eine erste Schicht von peritonealer Membran und eine zweite Schicht von peritonealer Membran umfasst.

7. Implantat nach Anspruch 6, worin sich das Stützelement zwischen der ersten Schicht von peritonealer Membran und der zweiten Schicht von peritonealer Membran befindet.

8. Implantat nach einem der Ansprüche 1 bis 7, worin das Stützelement eine oder mehrere Öffnungen umfasst, die sich zumindest teilweise dort hindurch erstrecken.

9. Implantat nach Anspruch 8, worin sich die Schicht von peritonealer Membran zumindest teilweise durch die Öffnung des Stützelements erstreckt, um die Schicht von peritonealer Membran mit dem Stützelement zu verbinden.

10. Implantat nach Anspruch 9, worin das Stützelement Mittel zur Haftung der Schicht von peritonealer Membran, die sich zumindest teilweise durch die Öffnung erstreckt, umfasst.

11. Implantat nach einem der Ansprüche 1 bis 10, worin die Schicht von peritonealer Membran Mittel zur Förderung des Einwachsens von Gewebe umfasst.

12. Implantat nach einem der Ansprüche 1 bis 11, worin die Form des Implantats vorbestimmt ist, um zur Form einer Bauchhöhle zu passen.

## Revendications

1. Implant pour réparation tissulaire comprenant une couche de membrane péritonéale et un élément de support pour renforcer la couche de membrane péritonéale, la couche de membrane péritonéale étant dérivée d'un segment de la paroi abdominale d'un vertébré à sang chaud, la couche de membrane péritonéale comprenant une membrane d'un xénogreffon dérivé d'un animal, la couche de membrane péritonéale comprenant une membrane d'un xénogreffon dérivé d'une source porcine, la couche de membrane péritonéale comprenant une membrane porcine dérivée d'une truie, l'implant pouvant être passé entre une configuration de mise en place et une configuration de déploiement, l'implant comprenant un moyen de contraindre l'implant à adopter préférentiellement la configuration de déploiement, le moyen de contrainte comprenant un matériau biodégradable.

2. Implant selon la revendication 1, dans lequel l'élément de support comprend un matériau biologique.

3. Implant selon la revendication 1 ou 2, dans lequel l'implant comprend un moyen de rattacher la couche de membrane péritonéale à l'élément de support.

4. Implant selon l'une quelconque des revendications 1 à 3, dans lequel l'implant comprend un laminé de la couche de membrane péritonéale et l'élément de support.

5. Implant selon la revendication 3 ou 4, dans lequel l'élément de support est au moins en partie encapsulé dans la membrane péritonéale.

6. Implant selon l'une quelconque des revendications 1 à 5, dans lequel l'implant comprend une première couche de membrane péritonéale et une seconde couche de membrane péritonéale.

7. Implant selon la revendication 6, dans lequel l'élément de support est situé entre la première couche de membrane péritonéale et la seconde couche de membrane péritonéale.

8. Implant selon l'une quelconque des revendications 1 à 7, dans lequel l'élément de support comprend une ou plusieurs ouvertures se prolongeant au moins en partie au travers de celui-ci.

9. Implant selon la revendication 8, dans lequel la couche de membrane péritonéale se prolonge au moins en partie au travers de l'ouverture de l'élément de support pour rattacher la couche de membrane péritonéale à l'élément de support.

10. Implant selon la revendication 9, dans lequel l'élément de support comprend un moyen d'agripper la couche de membrane péritonéale se prolongeant au moins en partie au travers de l'ouverture.

11. Implant selon l'une quelconque des revendications 1 à 10, dans lequel la couche de membrane péritonéale comprend un moyen de favoriser la croissance tissulaire.

12. Implant selon l'une quelconque des revendications 1 à 11, dans lequel la forme de l'implant est prédéfinie pour être adaptée à la forme d'une cavité abdominale.
